# EUROPÄISCHE PATENTSCHRIFT

(11) **EP 0 874 914 B1**
(45) Veröffentlichungstag und Bekanntmachung des Hinweises auf die Patenterteilung: **07.09.2005**
(21) Anmeldenummer: 97901033.7
(22) Anmeldetag: 15.01.1997
(51) Int. Cl.: C12Q 1/68, C12N 9/12

(54) **VERFAHREN ZUR AMPLIFIKATION VON NUKLEINSÄURE**
PROCESS FOR THE AMPLIFICATION OF NUCLEIC ACID
PROCEDE D'AMPLIFICATION D'ACIDE NUCLEIQUE

(30) Priorität: 16.01.1996 DE 19601385
(43) Veröffentlichungstag der Anmeldung: 04.11.1998
(73) Patentinhaber: MÜLLER, Manfred W., 3400 Klosterneuburg (AT)
(72) Erfinder: MÜLLER, Manfred W., 3400 Klosterneuburg (AT)
(74) Vertreter: Grünecker, Kinkeldey, Stockmair & Schwanhäusser Anwaltssozietät
(86) Internationale Anmeldenummer: PCT/EP1997/000160
(87) Internationale Veröffentlichungsnummer: WO 1997/026368

(56) Entgegenhaltungen:
- EP-A- 0 645 449
- WO-A-92/13088
- DE-C- 4 319 468
- WINNACKER E.-L.: "From genes to clones" 1987 , VCH , WEINHEIM/FRG XP002030912 siehe Seite 78 - Seite 79

## Beschreibung

Die vorliegende Erfindung betrifft ein Verfahren zur Amplifikation von Nukleinsäure sowie einen Kit für die Amplifikation von Nukleinsäure.

Eine Voraussetzung für die Analyse, insbesondere die Sequenzanalyse, von Nukleinsäuren ist, daß die zu analysierende Nukleinsäure in ausreichenden Mengen verfügbar ist. Insbesondere bei Nukleinsäuren in sehr niedriger Konzentration stellt deren Sequenzanalyse ein technisches Problem dar. Der Stand der Technik kennt zahlreiche Verfahren, um seltene, d.h. in niedriger Konzentration vorkommende Nukleinsäuren, vor der Analyse zu amplifizieren.

Besonders die Analyse der 5'-terminalen, kodierenden Region von RNA-Molekülen bereitet Schwierigkeiten. Eine in jüngster Zeit weit verbreitete Technik zur Amplifikation von Nukleinsäuren basiert auf der Polymerasekettenreaktion (der sogenannten PCR-Technik) zur in vitro-Amplifikation von DNA (vgl. Sambrook et al., Molecular Cloning, Laboratory Manual, 2. Auflage, Kapitel 14).

Soll die Struktur von RNA aufgeklärt werden, so ist vor der eigentlichen Amplifikationsreaktion eine cDNA von der zu analysierenden RNA herzustellen. Dabei tritt häufig das Problem auf, daß das 5'-Ende der RNA in geringer Frequenz in cDNA transkribiert wird und somit einer Sequenzanalyse schwerer zugänglich ist als weiter 3'-gelegene RNA-Abschnitte.

Der Stand der Technik beschreibt einige Möglichkeiten, um das 5'-Ende von RNA mit Hilfe der PCR-Technik zu amplifizieren. Gemeinsam ist den meisten dieser Verfahren, daß der von der RNA hergestellte erste cDNA-Strang an seinem 3'-Ende durch das Anfügen von weiteren Nukleotiden, das sogenannte "Tailing", verlängert wird, bevor der einzelsträngige cDNA-Strang zu der doppelsträngigen cDNA ergänzt wird.

Als eine Möglichkeit zum 3'-Tailing von DNA ist das Anhängen eines Oligonukleotidfragments an das freie 3'-Ende von cDNA oder das Anpolymerisieren von Desoxyribonukleotiden an das freie 3'-Ende beschrieben worden.

Diese bekannten Tailing-Verfahren haben jedoch den Nachteil, daß die Reaktion schwierig zu steuern ist, die Ausbeuten an 3'-verlängerten Molekülen teilweise den Anforderungen nicht genügen sowie unerwünschte Nebenprodukte entstehen, indem beispielsweise mehrere Oligonukleotide miteinander ligiert werden und so die 3'-Enden verschiedener DNA-Moleküle nicht gleichmäßig verlängert werden.

Der vorliegenden Erfindung lag das technische Problem zugrunde, ein weiteres Verfahren zur Amplifikation einer Nukleinsäure anzugeben, bei dem die aus dem Stand der Technik auftretenden Nachteile nicht beobachtet werden, und das insbesondere eine hohe Effizienz bei der 3'-Verlängerung von Nukleinsäuren gewährleistet.

Das obige Problem wird erfindungsgemäß gelöst durch ein Verfahren, bei dem das Tailing der Nukleinsäure dadurch erfolgt, daß die zu amplifizierende Nukleinsäure mit einem Ribonukleotid in der Anwesenheit von Terminaler Transferase (auch terminale Desoxynukleotidyl-Transferase (TdT) genannt) umgesetzt wird.

Dieses Verfahren, im folgenden auch CRTC (kontrolliertes Ribonukleotid-Tailing von cDNA) genannt, nützt die äußerst wirksame und gut kontrollierbare RNA-polymerisierende Aktivität der Terminalen Transferase unter üblichen Tailing-Bedingungen aus. Die üblichen Tailing-Bedingungen sind z.B. beschrieben in Deng G.R. und Wu, R. (1983), Methods in Enzymology 100: 96-116 und Roychoudkurg R. et al., (1976), Nucleic Acids Res. 3: 863-877. Diese TdT-Aktivität fügt eine begrenzte Anzahl von Ribonukleotiden, welche als Triphosphate (rNTPs) eingesetzt werden, an das 3'-Ende einer einzelsträngigen oder doppelsträngigen Nukleinsäure, vorzugsweise einer DNA, an. Vorzugsweise werden 2-4 Ribonukleotide anpolymerisiert.

Im Gegensatz zu der Primer-abhängigen DNA-Polymeraseaktivität von TdT, die den wiederholten Einbau von Desoxyribonukleotiden (dNTPs) an den 3'-Terminus einer einzel- oder doppelsträngigen DNA katalysiert, dem Desoxyribonukleotid-Tailing, ist das Ribonukleotid-Tailing einer einzelsträngigen DNA weder beeinflußt durch die Art der angefügten Basen (rGTP, rATP, rUTP bzw. rCTP) noch durch die Art des 3'-terminalen Nukleotids des DNA-Primers (G_{OH}, A_{OH}, T_{OH} oder C_{OH}). Unter Standardbedingungen wird eine Effizienz von mehr als 95 % mit dem erfindungsgemäßen Ribo-Tailing von cDNAs erreicht. Ganz besonders bevorzugt für das Ribo-Tailing ist rGTP.

Die Erzeugung eines kurzen homopolymeren Anhangs aus Ribonukleotiden an dem 3'-Ende der Nukleinsäure erlaubt die unidirektionale Ligierung dieser Nukleinsäure, beispielsweise eines ersten cDNA-Strangs, an eine linearisierte Plasmid-DNA mit einem komplementären 3'-überhängenden Ende in der Anwesenheit einer Ligase, vorzugsweise von T4-DNA-Ligase. Das erfindungsgemäße Verfahren macht somit das Anfügen eines Abschnitts homopolymerer Desoxyribonukleotide überflüssig, das oft schwierig zu optimieren und zu kontrollieren ist und häufig zu unspezifischen PCR-Produkten führt, und das erfindungsgemäße Verfahren vermeidet auch die Probleme, die auftreten bei dem Ligieren eines einzelsträngigen Oligonukleotids (auch Anker genannt) an das 3'-Ende von cDNAs durch T4-RNA-Ligase.

In dem erfindunsgemäßen Verfahren wird vorzugsweise eine einzelsträngige DNA eingesetzt, und ganz besonders eine cDNA, wobei diese sich in bevorzugter Weise von einer Poly (A)⁺-RNA ableitet.

Bei einer bevorzugten Ausführungsform des erfindungsgemäßen Verfahrens wird nach dem Tailingschritt an das 3'-Ende des verlängerten Nukleinsäuremoleküls ein weiteres Nukleinsäuremolekül gebunden.

Das weitere Nukleinsäuremolekül ist vorzugsweise eine doppelsträngige DNA und besitzt ein 3'-überhängendes Ende, welches komplementär ist zu dem 3'-Ende des durch die Ribonukleotide verlängerten Nukleinsäuremoleküls. Diese Komplementarität zwischen dem weiteren Nukleinsäuremolekül und dem verlängerten Nukleinsäuremolekül begünstigt das zielgerichtete Anbringen des weiteren Nukleinsäuremoleküls durch dessen Hybridisieren an das 3'-Ende des verlängerten Nukleinsäuremoleküls. Dieser Vorgang wird auch als "Tagging" bezeichnet.

Bei einer besonders bevorzugten Ausführungsform ist das weitere Nukleinsäuremolekül ein DNA-Vektor oder ein Adaptermolekül (auch Linker genannt), welches vorzugsweise gleichzeitig eine Erkennungssequenz für mindestens ein Restriktionsenzym enthält.

Weiterhin ist es bevorzugt, daß das weitere Nukleinsäuremolekül an das 3'-Ende des verlängerten Nukleinsäuremoleküls nicht nur hybridisiert, sondern auch ligiert wird, vorzugsweise mit T4-DNA-Ligase.

Das weitere Nukleinsäuremolekül ist bei einer besonders vorteilhaften Ausführungsform gleichzeitig der Primer für die Synthese eines zweiten DNA-Strangs, der den ersten DNA-Strang komplementär ergänzt. Die Synthese des komplementären Stranges kann vor der eigentlichen PCR-Amplifikation erfolgen, so daß zunächst eine doppelsträngige Nukleinsäure erhalten wird, die dann mit der herkömmlichen PCR-Technik amplifiziert wird. Jedoch kann die Synthese des komplementären Strangs (Zweitstrangsynthese) gleichzeitig mit der PCR-Amplifikation erfolgen.

Das erfindungsgemäße Verfahren eignet sich für sämtliche Verfahren, bei denen DNA-Adaptoren verwendet werden und/oder ein dNTP-Tailing mittels TdT erfolgt (wie beispielsweise in Verfahren unter Verwendung des PCR-Select-cDNA-Subtraction-Kits von Clontech, veröffentlicht in CLONTECHniques, Oktober 1995), besonders eignet es sich aber zur Sequenzanalyse von RNA, insbesondere zum Erfassen der 5'-Sequenz einer selten vorkommenden RNA.

Dabei ist es zunächst erforderlich, daß von der zu analysierenden RNA ein erster cDNA-Strang hergestellt wird. Dies kann durch die im Stand der Technik bekannten Verfahren erfolgen. Der erhaltene einzelsträngige cDNA-Strang wird dann amplifiziert durch die Maßnahmen, wie oben im einzelnen erläutert. Die erhaltene amplifizierte doppelsträngige DNA kann anschließend herkömmlichen Verfahren zum Sequenzieren von DNA unterzogen werden.

Des weiteren erlaubt die vorliegende Erfindung die Bereitstellung eines Kits für die Amplifikation einer beliebigen Nukleinsäure, wobei der Kit mindestens ein Ribonukleotid enthält, vorzugsweise ausgewählt aus rGTP, rATP, rUTP und rCTP, sowie die Terminale Transferase.

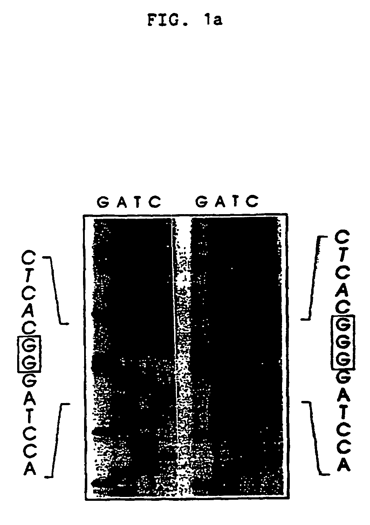

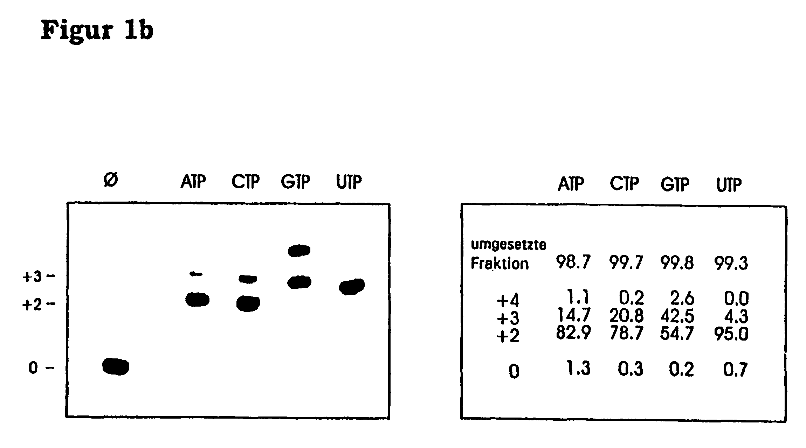

Figur 1 zeigt das Ergebnis eines erfindungsgemäßen Ribonukleotid-Tailings von einzelsträngiger DNA.

0,02 pMol eines 5'-terminal mit ³²P markierten DNA-Oligonukleo-tids (21-mer; 5'-GTTACCATTTTAATACACTTG) wurden inkubiert mit rNTPs (ATP, CTP, GTP oder UTP) in der Anwesenheit von TdT unter Reaktionsbedingungen wie in der folgenden Beschreibung näher ausgeführt. Die Produkte wurden auf einem 20% Poly(A)crylamid/8M Harnstoffgel fraktioniert, autoradiographiert und quantifiziert mit Hilfe eines Phosphorlmager (Modell 400B, Molecular Dynamics).

Spezifischer, Figur 1a zeigt die Analyse der 5'-terminalen Sequenzen von RNAs, umfassend das kontrollierte Ribonukleotid-Tailing von cDNAs, Tagging, PCR-Amplifikation und das Dideoxy-Standardsequenzierverfahren der erhaltenen und klonierten PCR-Produkte. Die Vektorsequenzen sind fett gedruckt gezeigt. Die cDNA-Sequenzen sind kursiv gezeigt. Die GG- und GGG-Nukleotide, die dem 3'-Terminus der cDNA-Sequenzen durch das TdT katalysierte Ribonukleotid-Tailing angefügt wurden, sind angegeben. Die Reinigung der biotinylierten PCR-Produkte, die in den unten näher beschriebenen Verfahren erzeugt wurden, wurde durchgeführt mit magnetischen Kügelchen, wie beschrieben von Hultmann, S., Staahl, E., Hornes, M. und Uhlen, L. (1989), Nucleic Acids Res. 17, 4937-4939. Nach einer Doppelspaltung mit SacI und EcoRI wurden die PCR-Produkte in die entsprechenden Restriktionsspaltstellen eines pBluescript II KS + Phagmids (Stratagene) eingefügt und dem Dideoxy-Standardsequenzierverfahren mit einem T7-Primer (5'-AATACGACTCACTATAG) in der Anwensenheit von Sequenase (Amersham Life Science) unterzogen.

Figur 1b zeigt die Prozentsätze an den nicht umgesetzten Fraktionen (0), von verlängerten Molekülen (+2 bis +4) und den gesamten Umsatz (umgesetzte Fraktion) abhängig von dem verwendeten rNTP. φ bedeutet die Kontrollreaktion in der Abwesenheit von rNTPs. Die Natur der Reaktionsprodukte, verlängert durch zwei (+2) oder drei (+3) Ribonukleotide, wurde bestätigt durch eine PCR-vermittelte Analyse der RNA-Sequenzen, wie beschrieben in Hetzer, M. und Mueller, M.W. (1993), Nucleic Acids Res. 21, 5526-5527.

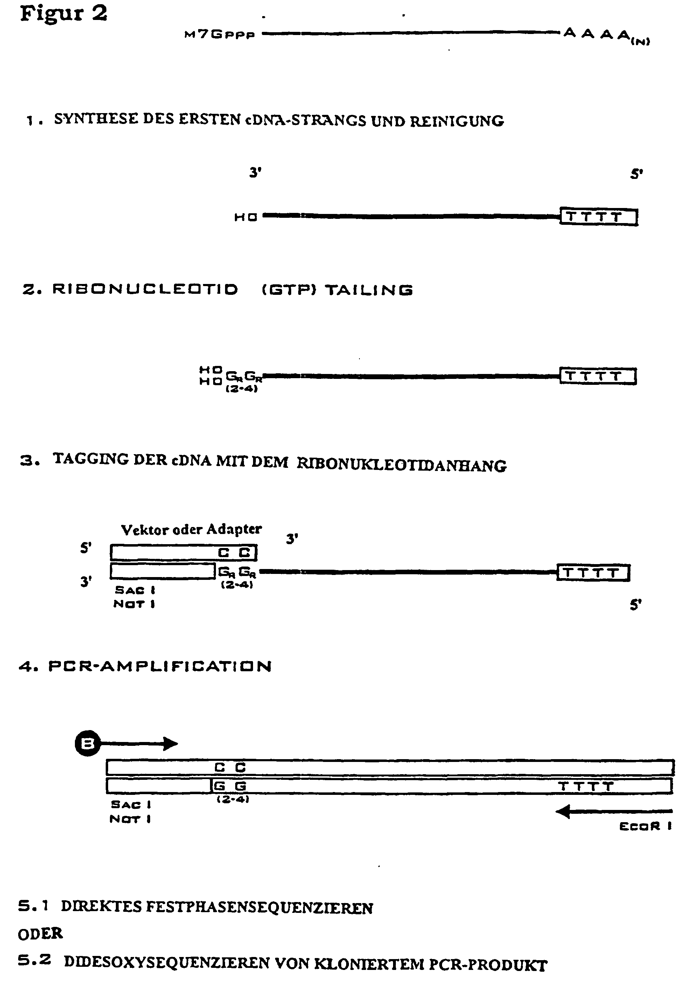

Figur 2 zeigt das Prinzip des CRTC bei dem PCR-vermittelten Klonieren und Sequenzieren von unbekannten 5'-Enden einer spezifischen mRNA. Der erste Schritt stellt die Erststrang-cDNA-Synthese von Gesamt-RNA oder Poly(A)⁺mRNA dar, und sie wird durchgeführ mit einem mRNA-spezifischen Primer, der 3' gelegen ist (eine bekannte Region in dem 3'-Teil der mRNA-Matrize ein Oligo(dT)Primer). Im zweiten Schritt werden die gereinigten cDNA-Produkte an ihren 3'-Enden durch das Anfügen von zwei bis vier Ribonukleotiden (rGTPs) modifiziert und im dritten Schritt an einen spezifischen doppelsträngigen DNA-Adaptor über einen komplementären 3'-Dinukleotidüberhang (CC) in der Anwesenheit von T₄-DNA-Ligase angebracht. Im vierten Schritt erfolgt die PCR-Amplifikation unter Verwendung eines adaptorspezifisches Primers (dieser ist 5'-terminal biotinyliert) und einem cDNA-spezifischen "nested" Primer, enthaltend eine gewünschte Restriktionsspaltstelle; im gezeigten Fall EcoRI). Die PCR-Amplifikation erzeugt ein einzigartiges PCR-Produkt, das geeignet ist zur direkten Festphasensequenzierung nach Hultmann et al., (1989), Nucleic Acids Res., 17: 4937-4939), oder für die Dideoxysequenzierung nach einem Klonierungsschritt unter Verwendung der Restriktionsspaltstellen, die aus dem DNA-Adaptor (SacI, NotI wie im Beispiel) oder von dem cDNA-Primer stammen.

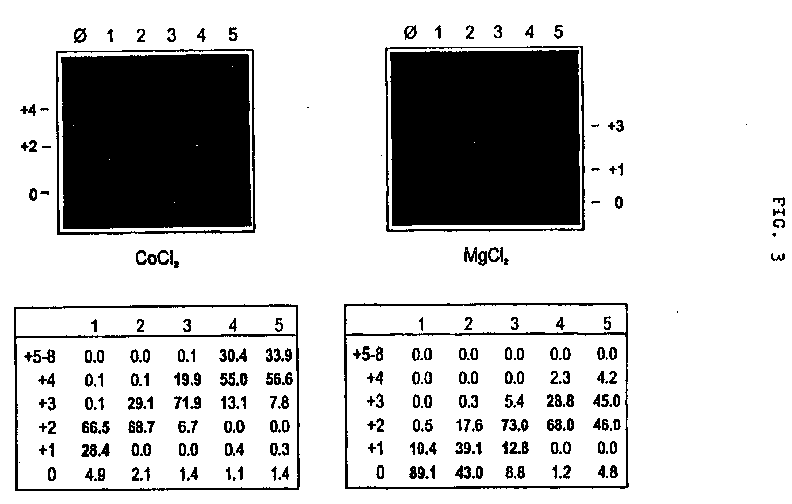

Figur 3 zeigt die Ergebnisse des rGTP-Tailings von cDNA unter verschiedenen Bedingungen. Es wurden CO²⁺ oder Mg²⁺ enthaltende Cacodylatpuffer verwendet. 0,005 µM eines am 5'-Terminus mit ³²P markierten DNA-Oligonukleotids (25-mer, 5' GTGAGACAAGTATAAG TATATTATT) wurden mit einem 10¹-fachen bis 10⁵-fachen Überschuß an rGTP als dem Substrat in CO²⁺ oder Mg²⁺ (1,5 mM) enthaltendem Cacodylatpuffer unter Enzymüberschuß bei 37°C für 60 Minuten in einem 10 µl Reaktionsansatz inkubiert. Die Produkte wurden auf einem 20% Poly(A)crylamidgel/8 M-Harnstoff Gel fraktioniert, autoradiographiert und quantifiziert (Phosphorlmager Model 400B, Molecular Dynamics). Die Konzentration der rGTP-Substrate (1 bis 5: 0,05 µM; 0,5 µM; 5 µM; 50 µM; 500 µM), die Prozentsätze an nicht umgesetzten Fraktionen (0) und der umgesetzen Fraktionen sind in Abhängigkeit des verwendeten Metallions angegeben. ∅ bedeutet die Kontrollreaktion in der Abwesenheit von rGTP.

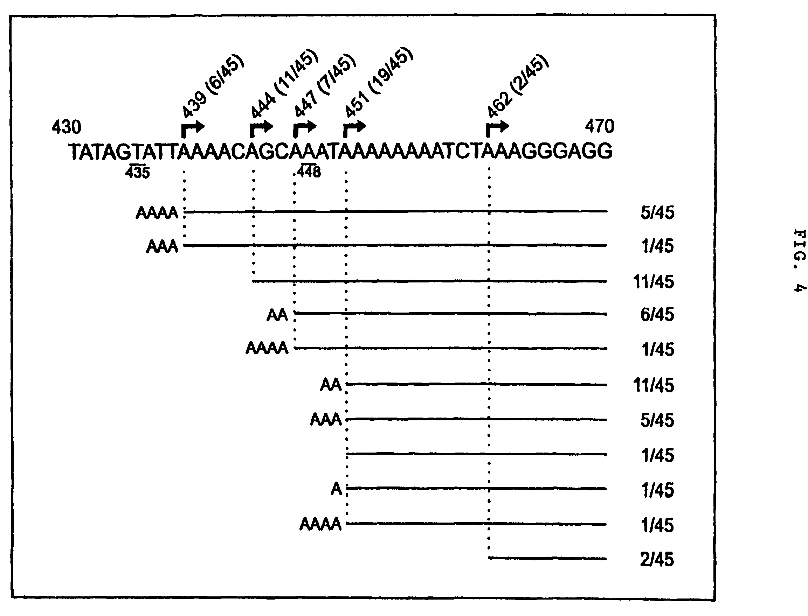

Figur 4 zeigt die Bestimmung des Transkriptionsstarts des DNA-Topoisomerase I-Gens von Hefe (*Saccharomyces cerevisiae*) durch CRTC-vermittelte Amplifikation von cDNA-Sequenzen. Zum Lokalisieren des Transkriptionsstarts des YSCTOPI-Gens (YSCTOPI; EC-Nr. 5.99.1.2), ausgehend von einer Poly(A)⁺mRNA-Population, wurden die folgenden Variationen unseres Standardprotokolls angewendet. (1) Poly(A)⁺mRNA (500 ng) vom Hefestamm DBY-746 (ClonTech) wurde einer Erststrang-cDNA-Synthese mit einem Primer, der spezifisch ist für den Minusstrang der DNA-Topoisomerase I (Position 941 bis 965), unterzogen. (2) Das Ribonukleotid-Tailing der cDNAs wurde durchgeführt mit rCTP (5 µM) in einer 50 µl Reaktion. (3) Ein Aliquot (10 bis 20%) der rC-verlängerten cDNA-Produkte wurde mit einem komplementären dG-Dinukleotid-3'-Überhang ligiert (tagged), wobei der Überhang durch Doppelspaltungen (BsgI x XbaI) des BSII/KS+IBsgI-Plasmids (2,5 fmol) erzeugt worden war. (4) Die mit den Vektoren versehenen cDNAs (10%) wurden direkt einer PCR-Amplifikation (94°C, 20 Sekunden; 67°C, 60 Sekunden; 40 Zyklen) unterzogen unter Verwendung des 5'-terminal biotinylierten, vektorspezifischen Primers (M13 revers; 25 pMol) und einem YSCTOPI-genspezifischen Primer (Position 706 bis 727; 25 pMol) umfassend eine EcoRI-Stelle an seinem 5'-Ende. Nach Spaltung mit EcoRI wurden die erhaltenen Fragmente in der EcoRI-Stelle eines pBluescript II KS + Phagemid (Stratagene) kloniert, und 45 Klone wurden durch das Dideoxysequenzierverfahren unter Verwendung eines automatischen DNA-Sequenzierers (LI-COR 4000L) analysiert.

Unter Anwendung dieses Verfahrens wurden 5 einzelne Transkriptionsstartstellen auf dem YSCTOPI-Gen (EC-Nr. 5.99.1.2) (Positionen 439 (6/45); 444 (11/45); 447 (7/45); 451 (19/45) und 462 (2/45)) charakterisiert. Die Abweichungen von der genomischen Sequenz (der Bereich von ein bis vier T-Resten, die dem kurzen Stück an rCMP-Anhang an dem 3'-Ende der cDNAs vorangestellt sind) sind als Reste A dargestellt. Die zwei möglichen Transkriptionsstartstellen, die zuvor von Thrash et al., Proc. Natl. Acad. Sci., USA, 82: 4374-4378 (1985), vorgeschlagen worden waren, aufgrund von theoretischen Überlegungen, sind unterstrichen.

Das hierin beschriebene CRTC durch terminale Transferase ist eine alternative Strategie bei auf Amplifikation beruhenden Ansätzen zur cDNA-Klonierung und mRNA-Sequenzanalyse. Das empfohlene Verfahren bietet mehrere Vorteile gegenüber den üblichen dNTP-Tailingreaktionen und bietet mehrere Verbesserungen, wenn es mit anderen Protokollen für die auf PCR basierender Analyse von cDNA-Sequenzen verglichen wird. Erstens, im Gegensatz zu dem Homopolymer-Tailing mit dNTPs durch terminale Transferase (Eschenfeldt et al., (1987), Meth. Enzymol. 152: 337-342) ist die CRTC leicht zu kontrollieren, selbst-beschränkend, da nur zwei bis vier Reste angefügt werden, und äußerst effizient. Unter den hierin empfohlenen Reaktionsbedingungen wird ein minimaler Einbau von 85% erreicht; typische Werte liegen in dem Bereich von mehr als 98%. Zweitens, weder das Ausmaß der Umsetzung (Umsetzungsrate) noch der Umfang der Verlängerung durch rNTPs (Anzahl der angefügten rNTPs) werden stark durch die als Substrat verwendeten Ribonukleotide beeinflußt; auch werden diese Parameter nicht stark durch die Art des terminalen Desoxynukleotids des DNA-Primers beeinflußt. Schließlich erlaubt die Verwendung eines kurzen homopolymeren Ribonukleotidanhangs an dem 3'-Ende der cDNA die unidirektionale Ligierung einer Erstrang-cDNA an eine doppelsträngige DNA (linearisierte Plasmid-DNA oder Adapter) mit einem komplementären 3'-Überhang in der Anwesenheit von T₄-DNA-Ligase. Dieses Verfahren beseitigt nicht nur die Notwendigkeit, homopolymere DNA-Anhänge bei auf Amplifikation beruhenden Ansätzen zu verwenden (RACE; rasche Amplifikation von cDNA-Enden; vgl. Frohman et al., (1988), Proc. Natl. Acad. Sci, USA 85: 8998-9002), was schwierig zu optimieren und zu kontrollieren ist und oft zu einem hohen Background an unspezifischen PCR-Produkten führt, sondern vermeidet auch die Probleme, die durch die Ligierung eines einzelsträngigen Ankeroligonukleotids an die 3'-Enden von cDNAs (SLIC; Einzelstrangligierung von cDNA-Enden) durch T₄-RNA-Ligase hervorgerufen werden (Vgl. Edwards et al., (1991), Nucleic Acids Res., 19: 5227-5232 und Troutt et al., (1992), Proc. Natl. Acad. Sci, USA 89: 9823-9825). Zusammenfassend kann man feststellen, daß im Vergleich zu RACE oder SLIC das erfindungsgemäße CRTC-Verfahren eine höhere Spezifität und Zuverlässigkeit bei PCR-vermittelten Analysen von mRNA-Sequenzen ermöglicht.

Unter den empfohlenen Reaktionsbedingungen für das Hompolymer-Tailing mit rNTPs ist das Ausmaß des cDNA-Tailings äußerst effizient (>98%) und selbstbeschränkend, wobei ein kontrollierter Einbau von zwei bis vier Ribonukleotiden an das 3'-Ende einer cDNA erfolgt. Das Ausmaß und der Umfang der Verlängerungsreaktion kann je nach Bedarf durch die Wahl der Konzentration der terminalen Transferase, der Konzentration der cDNA-Enden in der Reaktion und der Konzentration der als Substrat verwendeten rNTPs variiert werden. Unter den Bedingungen eines molaren Überschusses an Enzym über die DNA-Enden wird die Reaktion von dem Enzym gesteuert, und die Konzentration der cDNA-Termini übt einen geringen Einfluß auf das Ausmaß der Reaktion aus (Vgl. Eschenfeldt et al., siehe oben). Somit beeinflußt die Konzentration der rNTPs bei einem Enzymüberschuß in dem Reaktionsansatz nur den Grad der Verlängerungsreaktion aber nicht das Ausmaß der Reaktion. Als eine Konsequenz kann, falls gewünscht, der Grad des rNTP-Einbaus bis auf ein Nukleotid verringert werden durch Erniedrigen der rNTP-Konzentration in der Reaktionsmischung.

Das erfindungsgemäße CRTC-Verfahren eröffnet weiterhin die Möglichkeit, die vollständige Sequenz einer Poly(A)⁺mRNA zu klonieren. Die Spezifität von Oligo(dT)Primern bei der cDNA-Synthese und der Primer bei der PCR bereitet oftmals Probleme. Die Schwierigkeiten stammen von der unterschiedlichen Länge der homopolymeren Poly(A)-Anhänge. Diese Probleme können teilweise gelöst werden unter Verwendung eines Oligo(dT)Primers mit einer zusätzlichen spezifischen Sequenz an dem 5'-Ende, wie es bei der "RACE-Amplifikation" von cDNA-Enden erfolgt (RACE; vgl. Frohmann et al., (1988), Proc. Natl. Acad. Sci, USA 85: 8998-9002). Die anschließende erneute Amplifikation beruht dann auf einem Primer, der gegen eine spezifische Sequenz gerichtet ist. Alternativ kann der Oligo(dT)Primer mit zwei degenerierten Positionen (3'(A, G, C, T)(A, G, C)) synthetisiert werden, wobei dies zu einer präziseren Hybridisierung an der Verknüpfungsstelle zwischen mRNA und Poly(A)⁺-Anhang bei der Erststrang-cDNA-Synthese führt. Eine Kombination der beiden obigen Alternativen steigert die Spezifität, und deshalb ist ein am 3'-Terminus degenerierter Oligo(dT)Primer, enthaltend zusätzliche spezifische Sequenzen an seinem 5'-Ende, bei der Erststrang-cDNA-Synthese empfehlenswert.

Die folgenden Beispiele erläutern die Erfindung:

Zur Analyse einer RNA kann von einer Poly(A)⁺-RNA mit einem 7- Methyl-GTP (M7-GPPP) ausgegangen werden.

Erster Schritt: Synthese eines ersten cDNA-Strangs und dessen Reinigung.

Die Synthese eines ersten cDNA-Strangs beruht auf einer bekannten spezifischen Sequenzinformation aus der 3'-Region der zu analysierenden RNA oder dem Poly(A)-Anhang, der bei den meisten mRNAs vorhanden ist. Es wird ein Sequenzspezifischer Primer oder ein Oligo(dT)Primer verwendet, um die cDNA-Synthese, basierend auf der mRNA-Matrize einzuleiten. Nach dem Abbau der mRNA-Matrize durch eine Alkalibehandlung wird die Reinigung der cDNA erreicht mit Hilfe einer Silikamatrix in einem Zentrifugiergefäß.

### Zweiter Schritt: Homoribonukleotid-Tailing

Das Homoribonukleotid-Tailing des ersten cDNA-Strangs durch TdT kann durchgeführt werden in der Anwesenheit eines beliebigen rNTP (rGTP, rATP, rUTP oder rCTP). Die Reinigung der 3'-verlän-gerten cDNAs und die Probengewinnung wird durchgeführt mit einer Silikamatrix in einer für die Zentrifugation geeigneten Säule.

### Dritter Schritt: Tagging der cDNA

Die einzelsträngige cDNA, die an ihrem 3'-Ende mit zwei bis vier Homoribonukleotiden versehen ist, wird kovalent ligiert mit einer linearisierten Vektor-DNA oder einem DNA-Adapter mit einem 3'-überhängenden Ende, das komplementär ist zu dem 3'-Ende der verlängerten DNA. Für die Ligierung wird T4 DNA-Ligase verwendet.

### Vierter Schritt: PCR-Amplifikation der cDNA

Die PCR-Amplifikation wird durchgeführt mit einem 5'-terminal biotinylierten, ersten Primer, der spezifisch ist für den Vektor oder den Adapter, und dem cDNA-Primer als zweiten Primer, der bei der Synthese des ersten cDNA-Strangs verwendet wurde und der in der Regel aus dem 3'-Bereich der RNA stammt. Gegebenenfalls kann an dessen Stelle auch ein anderer, aus dem 3'-Bereich stammender Primer (nested Primer) verwendet werden. Als Polymerase wird DNA-Polymerase verwendet, vorzugsweise Taq-Polymerase.

### Fünfter Schritt: Sequenzieren der amplifizierten DNA

Es kann beispielsweise die direkte Festphasensequenzierung des amplifizierten Produkts mit Hilfe von magnetischen Kügelchen als festem Träger (Hultmann, S., Staahl, E., Hornes, M. und Uhlen, L. (1989), Nucleic Acids Res. 17, 4937-4939) mit einem cDNA-spezifischen Primer in der Anwesenheit von T7 DNA-Polymerase erfolgen. Alternativ kann das Sequenzieren nach dem Dideoxy-Verfahren durchgeführt werden und zwar nach der Reinigung des PCR-Produkts oder nach einem Klonierungsschritt, bei dem das Produkt in die gewünschte Orientierung in einen Vektor eingebracht wird.

Das folgende Beispiel beschreibt ausführlich die Sequenzanalyse des 5'-Anteils eines RNA-Moleküls (403 Nukleotide lang), das durch in-vitro Transkription mit T7 RNA Polymerase hergestellt worden war.
*(1) Synthese des ersten cDNA-Strangs und Aufreinigung der Probe:* aus Gel extrahierte ssRNAs (0,1 pMol), hergestellt durch in-vitro Transkription eines PCR-Fragments enthaltend einen T7 Promoter, wurden einer cDNA-Synthese mit einem Sequenz-spezifischen Primer aus der 3'-Region der RNA-Matrize (S3: 5'-GGTATATGT TATATATAAAC) für 15 min bei 37°C unterzogen, wobei 10 Einheiten SuperScript Reverse Transkriptase (RT; Gibco BRL) in einem 20 µl Reaktionsansatz, enthaltend 50 mM Tris-HCl (pH 8,3), 75 mM KCl, 3 mM MgCl₂, 5 mM DTT, 0,3 mM dNTPs, verwendet wurden. Direkt folgend auf die Synthese des ersten Strangs wurde die mRNA-Matrize unter alkalischen Bedingungen abgebaut (50 mM Na₂CO₃/NaHCO₃, pH 9,5; 30 min, 95°C). Die Reinigung des cDNA-Produkts und die Wiedergewinnung der Probe in Wasser (QD) wurde durchgeführt mit einer Säule GlassMAX™ (Gibco BRL), die zur Zentrifugation geeignet ist.
*(2) Ribonukleotid-Tailing von cDNAs:* 0,01 pMol des gereinigten cDNA-Produkts wurden für 60 min bei 37°C in der Anwesenheit von 5 Einheiten TdT (Boehringer Mannheim) und rGTP (Pharma-cia) inkubiert. Der Inkubationsansatz hatte ein Volumen von 10 µl und enthielt 200 mM Kaliumcacodylat, 25 mM Tris-HCl, 0,25 mg/ml BSA, pH 6,6 (TdT-Buffer, Boehringer Mannheim), 1,5 mM CoCl₂, 0,5 µM rGTP. Die Reinigung der am 3'-Ende verlängerten cDNA wurde durchgeführt durch eine EtOH/NaAc (0,3 M) Präzipitation.
*(3) Tagging einer mit rG-verlängerten cDNA mit einem linearisierten Plasmidvektor mit einem komplementären 3'-über-hängenden dC-Dinukleotid mit Hilfe von T4 DNA-Ligase:* 2,5 fMol der rGTP-verlängerten cDNA wurden für 6 Std. bzw. über Nacht bei 16°C inkubiert mit 2,5 fMol eines mit Bsgl und EcoRI gespaltenen Derivats (BSII/KS+/BsgI) eines pBlue-script II KS + Phagmids (Stratagene) in der Anwesenheit von 1 Einheit T4 DNA-Ligase (Boehringer Mannheim) in einem Endvolumen von 20 µl DNA-Ligasepuffer gemäß Herstellerangaben (Boehringer Mannheim).
   Das BSII/KS+/BsgI-Derivat des Phagmids (Stratagene) wurde hergestellt durch Oligonukleotid-vermittelte Mutagenese durch Ersetzen des HindIII x PstI-Fragments in der Klonierungsstelle des Phagmids durch das 31-mer mit der Sequenz 5'-AGCTTGATATC *GAATTC*GTGCAGATACTGCA (die BsgI-Stelle ist unterstrichen; die EcoRI-Stelle ist kursiv gezeigt). Das BSII/KS+/BsgI-Phagmid erlaubt die Blauweiß-Selektion; die Spaltung mit BsgI erzeugt eine einzige Spaltstelle mit überhängenden Enden abwärts (N16/N14) von der BsgI-Erkennungsstelle, wobei ein 3'-überhängendes Dinukleotid (3'-CC) an der SmaI-BamHI-Stelle erzeugt wird.
*(4) PCR-Amplifikation der cDNA-Produkte aus (3):* 2 µl der ligierten Mischung wurden direkt der PCR-Amplifikation unter Verwendung der Standardtechniken (94°C, 20 s; 58°C, 20 s; 72°C, 40 s; 35 Zyklen) in einem 50 µl Reaktionsansatz unterzogen, wobei 1 Einheit Taq DNA-Polymerase (Boehringer Mannheim) und Pufferbedingungen, wie vom Hersteller empfohlen (Boehringer Mannheim), verwendet wurden. Als amplifizierte PCR-Produkte wurden erhalten: 215 bp bis 216 bp Fragmente. Die verwendeten PCR-Primer waren: ein BSII/KS+/ BsgI spezifischer Minusstrangprimer (M13-20 PCR; 5'-GACGTT GTAAAACGACGGCCAGT; 26 pmol; 5'-terminal biotinyliert); ein cDNAspezifischer, Plusstrangprimer (S3-PCR: 5'-AATAA*GAATTC* GCATGTCTAATTAGGTATATGTTATATATAAAC; 25 pMol) enthaltend eine EcoRI-Stelle (kursiv gedruckt) und teilweise komplementär (unterstrichen) zu der 3'-Region der cDNA-Sequenz des zweiten Strangs.
   *(5.1) Direkte Festphasensequenzierung der PCR-Produkte:* Die Reinigung der PCR-Produkte und das Sequenzieren nach dem Dideoxyverfahren unter Verwendung magnetischer Kügelchen als festem Träger wurde durchgeführt wie beschrieben in Hultmann, S., Staahl, E., Hornes, M. und Uhlen, L.(1989), Nucleic Acids Res. 17, 4937-4939. Die verwendeten Sequenzierprimer waren: S3: komplementär zu der 3'-Region des zweiten cDNA-Strangs.
   *(5.2) Dideoxysequenzierung von klonierten PCR-Produkten:* Die Reinigung der PCR-Produkte unter Verwendung magnetischer Kügelchen wurde durchgeführt wie beschrieben von Hultmann et al., siehe oben. Nach einer Doppelspaltung mit SacI x EcoRI wurden die PCR-Produkte in die entsprechenden Restriktionsspaltstellen eines pBluescript II KS + Phagmids (Stratagen) eingeführt und dem Dideoxy-Standardsequen-zierverfahren mit einem T7 Primer in der Anwesenheit von Sequenase (Amersham Life Science) unterzogen (siehe auch Fig. 1)

Der Umfang des Einbaus von zwei (13/20), drei (7/20) oder vier (0/20) rGMP-Resten an die 3'-Enden der cDNAs korrelierte gut mit den Werten, die bei den rGTP-Tailingreaktionen mit einzelsträngigen DNA-Primern in der Anwesenheit von 0,5 µM Substrat beobachtet wurden (+2, 68,7%; +3, 29,1%; siehe auch Figur 3).

### Verfahren zum kontrollierten Ribonukleotid(rGTP)-Tailing von cDNA mit terminaler Transferase

0,01 bis 0,05 pMol der gereinigten (GlassMAX™ spin column, Gibco BRL) Erststrang-cDNA wurden für 60 min bei 37°C in der Anwesenheit von 5,0 Einheiten TdT (Boehringer Mannheim) und rGTP (Pharmacia) in folgendem 10 µl Reaktionsansatz inkubiert: 200 mM Kaliumcacodylat, 25 mM Tris-HCl, 0,25 mg/ml BSA, pH 6,6 (TdT-Puffer, Boehringer Mannheim), 1,5 mM CoCl₂, 5 µM rGTP. Diese empfohlenen Bedingungen (Cacodylatpuffer enthaltend CoCl₂, terminale Transferase im molaren Überschuß über die Konzentration der cDNA-Termini, 10³-facher Überschuß von rGTP über cDNA 3'-Enden) sind am besten geeignet für den Einbau von zwei bis vier rGMPs an das 3'-Ende der einzelsträngigen DNA (Ausmaß der Umsetzung: 98,6%; Verlängerung durch die Reaktion: +1 (0,0%); +2 (6,7%); +3 (71,9%); +4 (19,9%)). Herabsetzen der rGTP-Konzentration in der Reaktionsmischung auf 0,5 µM fördert den Einbau von zwei bis drei rGMPs (Ausmaß der Umsetzung: 97,9%; Verlängerung durch die Reaktion: +1 (0,0%); +2 (68,7%); +3 (29,1%); +4 (0,1%) siehe auch Figur 3). Für die meisten Anwendungen werden rGTP-Konzentrationen in dem Bereich von 0,5 µM bis 5,0 µM empfohlen. Das gleiche Protokoll kann im wesentlichen für alle anderen rNTPs als Substrat verwendet werden. Die Reinigung der am 3'-Ende mit Ribonukleotiden verlängerten cDNA wird mit EtOH/NaAc (0,3 M) Fällung oder alternativ über eine GlassMAX™ spin column (Gibco BRL) bewerkstelligt.

### Kontrollierter Einbau von Ribonukleotidhomopolymeren and das 3'-Ende von cDNAs

In einem ersten Schritt bei dem kontrollierten Tailing von cDNA-Enden mit Ribonukleotiden wurde ein 5'-terminal mit ³²P markiertes Desoxyoligonukleotid (21-mer) für den Einbau einer begrenzten Anzahl an rNTP-Substraten verwendet. 0,02 pMol dieses DNA-Primers wurden in einem Cacodylatpuffer [(CH₃)₂ ASO₂], enthaltend COCl₂, mit 5 µM rNTPs (rATP, rCTP, rGTP oder rUTP) in der Anwesenheit von TdT bei 37°C für 60 Minuten unter Standardinkubationsbedingungen inkubiert, wie für das dNTP-Tailing beschrieben. Wie aus der Figur 1b ersichtlich, katalysiert TdT unter den für das dNTP-Tailing empfohlenen Bedingungen (Boehringer Mannheim) einen äußerst effizienten und kontrollierten Einbau der rNTP-substrate an das 3'-Ende eines einzelsträngigen DNA-Primers. In sämtlichen Testansätzen wurde eine gesamte Umsatzrate von über 98% für das Anfügen von zwei bis vier rNMPs erhalten. Die Art der um zwei (+2) und drei (+3) Ribonukleotide verlängerten Reaktionsprodukte wurde durch PCR-Analyse von RNA-Sequenzen bestätigt, wie von Hetzer M. und Müller M.W. (1993), Nucleic Acids Res. 21: 5526-5527 beschrieben.

Bei den Tailingreaktionen unter Verwendung von dNTPs als Substrat ist das Ausmaß und der Grad der Verlängerung durch die Desoxynukleotide eine Funktion der Konzentration von Enzym, der Konzentration der cDNA-Termini, der Konzentration der gewählten dNTP, des Puffers und des Metallions (siehe Chirpich, T.P. (1977), BBRC 78: 1219-1226, Eschenfeldt W.H. et al. (1987), Meth. Enzymol. 152: 337-342). Unter den Bedingungen eines Enzymüberschusses beeinflußt die Konzentration der cDNA-Termini das Ausmaß der Reaktion nur wenig (Eschenfeldt et al., s.o.) und die Reaktion wird maßgeblich von dem Enzym gesteuert. Unter speziellen Kalium-Cacodylat-Pufferbedingungen für das Ribotailing der cDNA-Enden sind weitere Faktoren, die das Ausmaß und die Länge des Reaktionsprodukts bestimmen, die rNTP-Konzentration und das Metallion.

Es wurde weiterhin der Einfluß verschiedener Konzentrationen an rGTP als das Substrat in CO²⁺- und Mg²⁺-haltigen Puffersystemen auf das Ausmaß und die Ausdehnung des Ribonukleotidtailings untersucht. In CO²⁺-haltigen Reaktionsmischungen erhöhte sich, wenn die Konzentrationen an rGTP von dem 10-fachen Überschuß über ssDNA-Enden bis zum 100-fachen Überschuß erhöht wurden, der Prozentsatz an erhaltenen Reaktionsprodukten und die Länge des Ribonukleotidanhangs (Figur 3). Bei einem 10²-fachen bis 10⁵-fachen Überschuß an rGTP wird nur die Länge des Anhangs beeinflußt, wobei der gesamte Umsatz von über 98% unverändert bleibt. In Mg²⁺-haltigem Puffer war ein 10³- bis 10⁵-facher Überschuß an rGTP über die DNA-Enden am besten für den Einbau von zwei bis drei rGMP-Resten geeignet.

Offensichtlich ist unter den speziellen empfohlenen Bedingungen (z.B. Cacodylatpuffer enthaltend CoCl₂, terminale Transferase im molaren Überschuß über die Konzentration der cDNA-Enden, 10²- bis 10³-facher Überschuß an rGTP über ssDNA) das Ribonukleotid-Tailing selbst-beschränkend, wobei zwei bis vier rGMPs eingebaut werden (Figur 3). Um diesen beschränkten Einbau ausführlicher zu untersuchen, wurden einzelsträngige DNA-Primer, die um 2, 3 oder 4 Ribonukleotide verlängert worden waren, aus dem Gel extrahiert und einer zweiten Runde des dNTP-Tailings unterworfen. Ein moderater und verzögerter wiederholter Einbau an Desoxynukleotiden wurde beobachtet, wenn Primer untersucht wurden, die durch zwei Ribonukleotide verlängert worden waren (17% nicht umgesetzt). DNA-Primer, die um drei (96% nicht umgesetzt) oder vier Ribonukleotide (99% nicht umgesetzt) verlängert worden waren, waren einem weiteren Einbau von dNTP-Substraten durch terminale Transferase praktisch nicht zugänglich. Umgekehrt, wenn die Ribonukleotid verlängerten DNA-Primer in der Anwesenheit von rNTP als dem Substrat untersucht wurden, war die Reaktion selbst-beschränkend, wobei als obere Grenze eine Gesamtzahl von vier Einbaureaktionen erfolgte. Diese Daten zeigen, daß unter den empfohlenen Reaktionsbedingungen DNA-Primer mit vier rNMPs am 3'-Ende inert gegenüber dem Einbau von weiteren Nukleotiden (dNTPs oder rNTPs) sind. Somit ist die Ribonukleotidverlängerung von cDNA-Enden leicht zu kontrollieren durch den sich selbst-beschränkenden Umfang des Einbaus der Nukleotide.

Weiterhin wurde untersucht, ob das Anfügen von Ribonukleotiden durch das 3'-terminale Nukleotid des einzelsträngigen DNA-Primers beeinflußt wird. Zu diesem Zweck wurden vier verschiedene Varianten eines 5'³²P-markierten DNA-Oligonukleotids (20-mer; 5' GGTATATGTTATATATAAAN; N=G, A, G oder C) analysiert in der Anwesenheit von rNTPs und TdT, wie oben beschrieben. Ein minimaler Einbau von mehr als 85% wurde erreicht, wenn DNA-Primer eingesetzt wurden, die mit dG-Enden versehen waren, wobei der Test in Anwesenheit von rUTP oder rGTP als dem Substrat durchgeführt wurde; typische Werte für das Anfügen von zwei bis vier rNMPs lagen in dem Bereich von 98%.

Alle Arten an doppelsträngigen DNA-Termini (3'-überhängende Enden, glatte Enden, 3'-kürzere Enden) können mit rNTPs als dem Substrat verlängert werden. Wie die dNTP-TailingReaktionen wurde die beste Reaktion mit 3'-überhängenden Enden erzielt.

Zusammenfassend kann man somit feststellen, daß diese Daten zeigen, daß - im Gegensatz zu der DNA-Polymerase-Aktivität der TdT - das Ribonukleotid-Tailing von einzelsträngigen DNAs sehr effizient verläuft und wegen dem beschränkten Ausmaß der Reaktion leicht kontrollierbar ist. Weiterhin ist das rNTP-Tailing nur leicht beeinflußt durch die Arten der angefügten Basen (rGTP, rATP, rUTP oder rCTP) und zumindest nicht stark beeinflußt durch die Art des 3'-terminalen Nukleotids des DNA-Primers.

### Bestimmen des Transkriptionsstarts des DNA-Topoisomerase-I-Gens der Hefe (Saccharomyces cerevisiae) durch CRTC, wobei von einer Poly(A)⁺mRNA ausgegangen wurde:

Das hierin beschriebene CRTC wurde auf mRNA des DNA-Topoisomerase-I-Gens von *Saccharomyces cerevisiae* (YSCTOPI; EC-Nr. 5.99.1.2; siehe Figur 4) angewendet. Zwei mögliche Transkriptionsstartstellen, an Position 435 und 448, sind von Thrash et al., (1985), Proc. Natl. Acad. Sci, USA, 82: 4374-4378) vorgeschlagen worden.

Unter Anwendung des erfindungsgemäßen Verfahrens wurden fünf einzelne Transkriptionsstartstellen des YSCTOPI-Gens charakterisiert (Positionen 439 (6/45), 444 (11/45), 447 (7/45), 451 (19/45) und 462 (2/45); siehe Figur 4). Neben dem Einbau von zwei bis drei rCMP-Resten an das 3'-Ende sämtlicher analysierter cDNAs enthielten mehrere Klone (31/45) Abschnitte von nicht kodierten T-Resten (eins bis vier) an dem C-Terminus. Offensichtlich beruht die Abweichung von der genomischen Sequenz auf der Wirkung von reverser Transkriptase am 3-Ende der cDNA-Produkte, wobei sie die terminalen mRNA-Sequenzen durch einen Rückfaltmechanismus unter Verwendung des cDNA-Terminus als einem Primer kopiert.

## Patentansprüche

1. Verfahren zur Amplfikation einer einzelsträngigen cDNA, die sich von einer Poly A⁺-RNA ableitet, umfassend die folgenden Schritte:
a) Umsetzen der zu amplfizierenden cDNA mit einem Ribonukleotid in Anwesenheit von Terminaler Transferase,
b) Verbinden des erhaltenen Umsetzungsprodukts am 3'-Ende mit einem weiteren doppelsträngigen Nukleinsäuremolekül mit einem 3' überhängenden Ende, welches komplementär ist zu dem 3'-Ende des Umsetzungsprodukts, wobei die weitere Nukleinsäure als Primer für die Synthese eines zweiten DNA-Strangs dient und dieser davon ausgehend synthetisiert wird.
c) Amplifizierung des in b) erhaltenen Doppelstranges mit PCR.

2. Verfahren nach einem der Ansprüche 1, **dadurch gekennzeichnet, dass** das weitere Nukleinsäuremolekül an das 3'-Ende des Umsetzungsprodukts ligiert wird, vorzugsweise in Anwesenheit von T4-DNA-Ligase.

3. Kit für die Amplifikation einer Nukleinsäure mittels PCR, umfassend: mindestens ein Ribonukleotid, Inkubationspuffer, Terminale Transferase und das doppelsträngige Nukleinsäuremolekül gemäß Anspruch 1b).

## Claims

1. A process for the amplification of a single-stranded cDNA which is derived from a poly A⁺-RNA, comprising the following steps:
a) reacting the cDNA to be amplified with a ribonucleotide in the presence of terminal transferase,
b) linking the obtained product of the reaction at the 3' end with a further doublestranded nucleic acid molucule with a 3' overhanging end, which is complementary to the 3' end of the product of the reaction, the further nucleic acid serving as a primer for the synthesis of a second DNA strand and the second DNA strand being synthesised starting therefrom.
c) amplifying the double strand obtained in b) with PCR.

2. The process according to claim 1, **characterised in that** the further nucleic acid molecule is ligated to the 3' end of the product of the reaction, preferably in the presence of T4 DNA ligase.

3. A kit for the amplification of a nucleic acid by means of PCR, comprising: at least one ribonucleotide, incubation buffer, terminal transferase and the doublestranded nucleic acid molecule according to claim 1b).

## Revendications

1. Procédé pour l'amplification d'un ADNc simple brin dérivé d'un ARN poly(A)+, comprenant les étapes suivantes :
a) conversion de l'ADNc avec un ribonucléotide en présence de transférase terminale
b) liaison du produit de la conversion obtenu à l'extrémité 3' avec une autre molécule d'acide nucléique double brin ayant une extrémité 3' proéminente qui est complémentaire de l'extrémité 3' du produit de la conversion, l'autre acide nucléique servant d'amorce pour la synthèse d'un deuxièmes brin d'ADN et ce dernier étant synthétisé à partir de là.
c) amplification par PCR du double brin obtenu en b).

2. Procédé selon la revendication 1, **caractérisé en ce que** l'autre molécule d'acide nucléique est ligaturée à l'extrémité 3' du produit de la conversion, de préférence en présence de la T4 ADN ligase.

3. Kit pour l'amplification par PCR d'un acide nucléique comprenant : au moins un ribonucléotide, un tampon d'incubation, une transférase terminale et la molécule d'acide nucléique double brin selon la revendication 1b).
